(19) Europäisches Patentamt / European Patent Office / Office européen des brevets

(11) **EP 2 338 413 B1**

(12) **EUROPEAN PATENT SPECIFICATION**

(45) Date of publication and mention
of the grant of the patent:
**06.08.2014 Bulletin 2014/32**

(51) Int Cl.:
***A61B 5/024*** *(2006.01)*

(21) Application number: **10196384.1**

(22) Date of filing: **22.12.2010**

(54) **Heart pulse monitors**

Herzfrequenzmonitore

Moniteurs à impulsions cardiaques

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2009 US 288872 P**

(43) Date of publication of application:
**29.06.2011 Bulletin 2011/26**

(73) Proprietor: **Stichting IMEC Nederland
5656 AE Eindhoven (NL)**

(72) Inventors:
• **Buxi, Dilpreet Singh
5629 LB, Eindhoven (NL)**
• **Penders, Julien
4020, Luik (BE)**

(74) Representative: **Mackett, Margaret Dawn et al
Gevers
Holidaystraat 5
1831 Diegem (BE)**

(56) References cited:
**FR-A1- 2 700 684     US-B1- 6 491 647**

## Description

The present invention relates to heart pulse monitors and is more particularly concerned with such monitors that can be worn at a convenient location by a user, for example, at the wrist.

Measuring the heart pulse and heart pulse rate is a topic of significant importance in personal health care, sports and stress monitoring. If a heart pulse rate or heart beat sensor can be packaged into a device, for example, a wristwatch, the sensor can be worn in a convenient position for the user. Additionally, such a wristwatch can also be worn for activities ranging from intensive sports to sleeping.

Heart pulse rate provides information about the general health status of an individual, and can indicate his/her general fitness and mental state, and his/her level of physical activity. In addition, the heart pulse rate can also be used to detect cardiac arrhythmia, such as bradychardia and tachycardia for instance. Heart pulse rate also provides information about other specific medical conditions such as the onset of an epileptic seizure, for instance.

Besides heart pulse rate monitoring, the heart rate variability (HRV) is also of significant interest. It is often claimed that the HRV carries information about autonomic regulation of cardiac activity. It is believed that, as a result of an arousing event, the heart rate will increase, in order to prepare the body for physical activity. In addition, it has been shown that the HRV has the potential to indicate the quality of a person's sleep. Other investigations highlight the importance of using the HRV as a parameter in calculating stress in drivers or military personnel. HRV also has been known to be affected by smoking, alcohol and caffeine consumption, as well as age and gender. In diseases, HRV is known to be affected by myocardial infarction, ventricular arrhythmias, hypertension, diabetes mellitus and heart failure. Typically, analysis of the HRV is done using a footprint, which is a recording of the heart rate (HR) and HRV over a period of 24 hours.

However, obtaining an accurate measurement of an active person's pulse rate at the wrist is a complex process. This is because of the presence of artefacts produced by body motion. These artefacts are detected by the heart pulse sensor as noise. In many cases, this noise can produce signals of sufficient amplitude to completely mask the heart pulse signal which is to be measured.

US Patent 5 807 267 discloses a heart rate monitor that is included in a wrist band. The heart rate monitor comprises two piezoelectric sensors arranged in a side-by-side configuration and is placed so that the sensors detect a pulse in an artery over which the sensors are placed. One of the sensors is used as a primary sensor and the other as a reference or background sensor. Signals generated by the reference or background sensor are digitally subtracted from signals generated by the primary sensor. The subtraction of the two signals reduces the effects due to motion of the user whilst using the heart rate monitor.

In the article entitled "Pulse Wave Sensor for Non-intrusive Driver's Drowsiness Detection", 31st Annual International Conference of the IEEE EMBS Minneapolis, Minneapolis USA, 2-6 September 2009, a heart pulse monitor device is disclosed for use on the steering wheel of a vehicle. The device consists of an array of pulse wave sensor units spaced around the periphery of the steering wheel. Each sensor unit comprise two piezoelectric sensors that are mounted on top of one another and spaced from one another by an isolation foam layer. Each piezoelectric sensor is in the form or a polyvinyl difluoride (PVDF) film layer mounted on a base material layer. The sensor unit is arranged such that one PVDF film layer is located against the steering wheel and the other PVDF film layer is uppermost. The isolating foam is located between the two base material layers. In use, the uppermost PVDF film layer detects a driver's heart pulse rate and the gripping force he/she applies to the steering wheel and any variations in the applied gripping force. The other PVDF film layer detects only the gripping force and any variations in the applied gripping force. Subtraction of signals detected by the PVDF film layers provides an indication of the alertness of the driver.

US Patent 6 491 647 describes a heart pulse monitoring device in which first and second sensors are used to provide a substantially artefact-free output signal. One sensor forms a measurement sensor and the other sensor forms a reference sensor. First and second output signals from respective ones of the first and second sensors are processed to provide an output indicative of heart pulse of a user.

Such a system is not robust or accurate enough to detect heart pulse rate information that can be used in medical applications.

It is therefore an object of the present invention to provide a heart pulse monitor device that can readily be worn by a user and from which relevant medical data can reliably be extracted.

In accordance with a first aspect of the present invention, there is provided a heart pulse monitoring device for determining the pulse of a user, the heart pulse monitoring device comprising:-. at least one sensor package mountable over a pulse location of the user, each sensor package including a first sensor element and a second sensor element, the first sensor element providing a first output signal indicative of the heart pulse of the user and the second sensor element providing a second output signal indicative of noise present in the first output signal, the first and second sensor elements comprise mechanical sensor elements mounted one on top of the other with a mechanically isolating material located between the first and second sensor elements, the mechanically isolating material isolating the first sensor element from the second sensor element; processing circuitry connected to each sensor package, the processing circuitry being operable to process the first and second output signals to provide a processed output signal indicative of the heart pulse of the user; characterised in that the first sensor element is mounted in a first packaging layer and the second sensor element is mounted in a second packaging layer, the properties of each packaging layers being different to one another; and in that the first packaging layer is adapted to electrically isolate the skin from the first sensor element, and the second packaging layer is adapted to prevent the second sensor element from being deformed due to heart pulse or heartbeat related vibrations.

The present invention has the advantage that it provides good pulse acquisition and good correlation between motion artefacts measured by both sensors in the sensor package. This is due to the packaging materials used in the construction of the heart pulse monitoring device.

One of the first and second sensor mechanical sensor elements comprises a primary sensor element and the other of the first and second sensor elements comprises a reference sensor element, the reference sensor element being mechanically isolated from the primary sensor element by the mechanically isolating material.

The material in at least one packaging layer can be represented as a mass-spring-damper arrangement.

Preferably, each of the first and second mechanical sensor elements comprises a piezoelectric sensor element. A preferred piezoelectric sensor element comprises a polyvinylidene fluoride film sensor element.

It is preferred that the polyvinylidene fluoride film sensor element has an acoustic impedance that is matched to that of the user's tissue. Alternatively, one piezoelectric sensor element has an acoustic impedance that is matched to that of the user's tissue and the other piezoelectric sensor element has an acoustic impedance that is mismatched to that of the user's tissue.

In one embodiment of the present invention, the processing circuitry may comprise a microprocessor for storing the first and second output signals and the processed signals. The processing circuitry may comprise filtering circuitry for filtering the first and output second signals. Such filtering circuitry may comprise, for example, Butterworth or Chebychev filters or any other suitable filtering technique. Adding filtering circuitry may improve the adaptive filtering algorithm.

Additionally, the processing circuitry may comprise amplifying circuitry, for example, a non-inverting amplifier, and/or analogue-to-digital converting circuitry for converting the output signal into a digital output signal.

Preferably, the device may further comprise displaying circuitry, such as a liquid crystal display, arranged for displaying the output signal.

Moreover, the processing circuitry may comprise an integrated radio and antenna for wireless transmitting data relating to the output signal to an external device, for example, a displaying device or a base station of a body area network.

Ideally, the first and second sensors comprise electronic lead attachments arranged for directing the first and second output signals to the processing circuitry.

The device may further comprise a housing enclosing the processing circuitry. The housing may comprise in a wrist watch case. In addition, an array of sensor packages may be attached to a wristlet and connected to the housing.

Additionally, an integrated heart rate monitor device in accordance with the present invention may be combined with an oximeter for measuring the oxygen level in the blood. The heart rate monitor device is arranged for measuring the pulse by a sensor that can obtain the artery palpitation typically in the radial or brachial artery in the arm. As electrical activity of the heart can be altered because of cardiac failure, the biopotential signal cannot be used for this purpose and therefore cardiac output is measured by mechanic sensing of the pulse. The oximeter, at the finger, is arranged for measuring the oxygen concentration in blood or arterial oxyhaemoglobin. This method is non-invasive invasive and has potentially a good sensitivity. Signals coming from both measuring devices can be processed and output by a central unit.

Such a combined device can for example be used for monitoring the outcome of a CPR manoeuvre and quantify its level of performance by measuring the circulation function by a pulse detector and respiratory function by the use of an oximeter. In an alternative embodiment, the oximeter may comprise a separate device to that of the heart pulse monitor. In this case, the two separate devices can communicate wirelessly with the processing of both devices being effected by the heart pulse monitor device.

In accordance with another aspect of the present invention, there is provided a method for detecting a heart pulse of a user using a heart pulse monitoring device as described above, the method comprising the steps of:- a) obtaining a first output signal from the first mechanical sensor element, the first output signal being indicative of the pulse of the user; b) obtaining a second output signal from the second mechanical sensor element, the second output signal being indicative of noise in the first output signal; c) removing noise from the first output signal using the second output signal; and d) providing a processed output signal that is representative of the pulse of the user.

If the heart pulse monitoring device comprises a plurality of sensor packages, the method comprises, for each sensor package, executing steps a) to d).

Additionally, the method may further comprise the step of transmitting data relating to the processed output signal for each sensor package to an external device.

output signal; c) removing noise from the first output signal using the second output signal; and d) providing a processed output signal that is representative of the pulse of the user.

If the heart pulse monitoring device comprises a plurality of sensor packages, the method comprises, for each sensor package, executing steps a) to d).

Additionally, the method may further comprise the step of transmitting data relating to the processed output signal for each sensor package to an external device.

In the method of the present invention, a first signal in response to a pulse at the pulse location and a second signal in response to artefact signals at the pulse location are measured. The method further comprises receiving and processing the first and second signals via processing circuitry. An output signal is produced which is representative of the difference between said first and said second output signals and is a function of the rate at which the first signal is received.

The method may further comprise filtering the first and second signals. In an embodiment, the method may comprise amplifying the first and second signals. In an embodiment, the method may comprise converting the output signal into a digital output signal. In an embodiment, the method may further comprise displaying the output signal. In another embodiment, the method may comprise wireless transmitting data relating to the output signal to an external device via an integrated radio and antenna.

The heart pulse monitoring device of the present invention senses a heart beat related signal at, for example, the wrist, which can be used for subsequent beat detection. The device focuses on the reduction of motion artefact while conditioning a signal for subsequent beat detection. Main related technical challenges include a low signal to noise ratio (SNR) at the measurement location and motion artefact. In order to mitigate the effects of these body artefacts, it is necessary to filter out and electronically cancel as much as possible of the noise signals occurring in the heart pulse frequency band as possible while retaining the desired pulse signal. The sensor package is designed such that adaptive filtering can succeed more, although adaptive filtering is not the only method for extracting the signal corresponding to the heart pulse.

The device also provides the possibility to measure heart rate in a more robust manner and in doing so, augment sports, sleep and stress monitoring. Furthermore, the device is refined packaging thereby increasing the level of integrity and autonomy of system.

For a better understanding of the present invention, reference will now be made, by way of example only, to the accompanying drawings in which:-

Figure 1 illustrates a block diagram of a sensor arrangement in accordance with the present invention;
Figure 2 illustrates a perspective view of a sensor arrangement in accordance with the present invention;
Figure 3 illustrates possible locations of sensor elements in relation to a person's wrist;

Figure 4 shows a sensor element illustrating possible directions of force and electric voltage generation within the sensor element;
Figure 5 illustrates a schematic diagram of a sensor package in accordance with the present invention;
Figure 6 illustrates output traces from the sensor of Figure 5;
Figure 7 illustrates possible lead attachments for the sensor package of Figure 5;
Figure 8 illustrates a schematic diagram of a piezoelectric sensor and a signal conditioning circuit;
Figures 9(a) and 9(b) respectively illustrate a bottom view and a side view of an example of a heart rate monitor device comprising an array of sensor packages in accordance with the present invention.

The present invention will be described with respect to particular embodiments and with reference to certain drawings but the invention is not limited thereto. The drawings described are only schematic and are non-limiting. In the drawings, the size of some of the elements may be exaggerated and not drawn on scale for illustrative purposes.

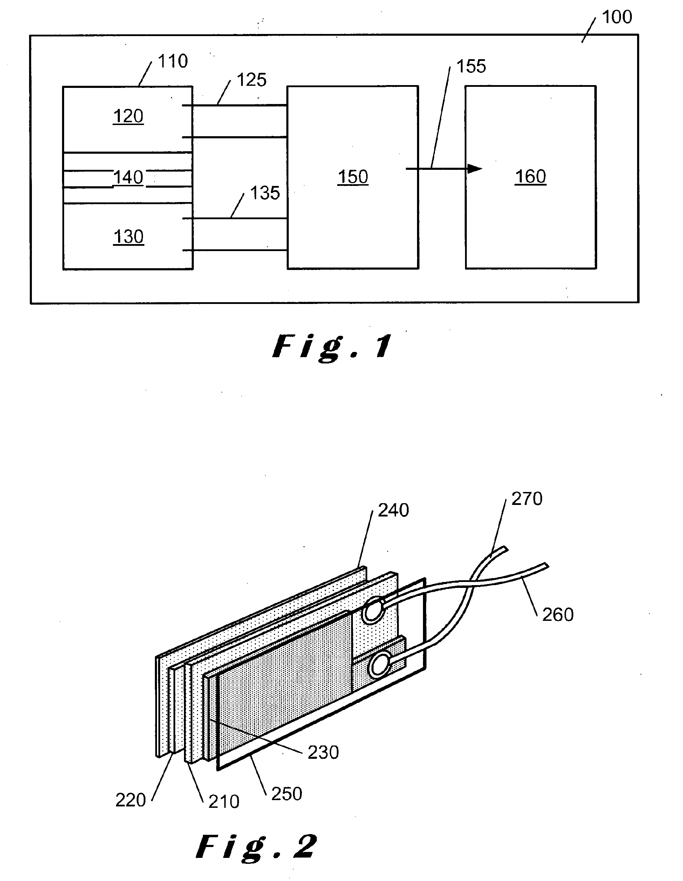

Referring initially to Figure 1, a heart pulse monitoring device 100 in accordance with the present invention is shown. The device 100 comprises a sensor package 110 that can be arranged adjacent a pulse location of the user, preferably on the wrist. The sensor package 110 comprises a first sensor 120 and a second sensor 130 spaced from the first sensor by a layer 140 of mechanically isolating material. The sensor package is connected to processing circuitry 150. The layer 140 of mechanically isolating material may be made of silicone or any other suitable compliant polymer foam or gel.

Each sensor 120, 130 comprises a mechanical sensor that provides respective first and second output signals 125, 135 that are passed to the processing circuitry 150 for processing. The first output signal 125 corresponds to a pulse and artefact signals at the pulse location. The second output signal 135 corresponds to artefact signals only at the pulse location.

As shown, the first and second sensors 120, 130 are mounted on top of one another with the layer 140 of mechanically isolating material located between the two sensors. The mechanical sensors 120, 130 are sensitive enough to detect the pulse at the wrist. It is preferred that the mechanical sensors 120, 130 comprise piezoelectric sensors, but it will be appreciated that any other type of suitable mechanical sensor can be used provided it is sensitive enough to detect a pulse generated by heart beats.

The processing circuitry 150 produces an output signal 155 from the first and second output signals 125, 135 which is indicative of the pulse rate of the user of the device 100.

The processing circuitry 150 operates to determine the difference between the first output signal 125 and the second output signal 135 to provide the heart pulse of the user. It may comprise a microprocessor that operates for both processing the first and second signals 125, 135 and storing the first and second output signals and the processed output signals. In addition, the processing circuitry may comprise filtering circuitry for filtering the first and output second signals using, for example, Butterworth or Chebychev filters or any other suitable filtering technique. If adaptive filtering is used when processing the first and second output signals, adding further filtering circuitry may improve the effectiveness of the adaptive filtering algorithm.

In addition, the processing circuitry may also include a non-inverting amplifier and an analogue-to-digital converter (ADC) for providing a digital output signal. It will be understood that the amplifier may be used alone or in combination with the ADC. Similarly, the ADC may be used alone or in combination with the amplifier.

Moreover, the processing circuitry may comprise an integrated radio and antenna for wireless transmitting data relating to the output signal to an external device, for example, a displaying device or a base station of a body area network. This allows data from the monitoring device relating to the heart pulse of a user to be transmitted to a central point for review and interpretation.

The heart pulse monitoring device 100 may also include a display 160 that displays the output signal 155 in a form that can readily be understood by the user, for example, in the form of beats per minute.

Turning now to Figure 2, a commercially available PVDF sensor 200 is shown. The sensor 200 comprises a PVDF film layer 210 that is located between two metallised coatings 220, 230. Protective coating layers 240, 250 are located on the outside of the two metallised coatings 220, 230. Leads 260, 270 provide connections to the sensor 200.

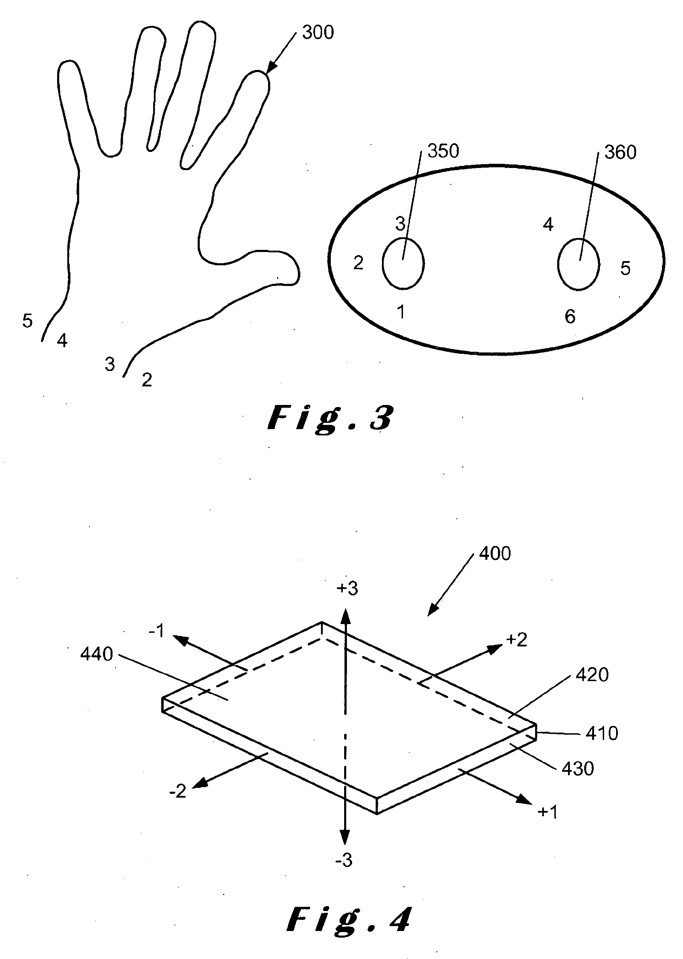

In Figure 3, possible pulse locations of a sensor in accordance with the present invention with respect to a user's wrist are shown. It will be appreciated that the heart rate pulse or heartbeat of a user must be detected at a suitable location for the sensor in accordance with the present invention to be able to detect and provide the desired signal output. A hand 300 is shown with locations '2', '3', '4', '5' located at the wrist.

Figure 3 also shows the possible locations where a pulse can be detected. Two pulse points 350, 360 are shown around which respective locations '1', '2', '3', '4', '5', '6' are shown.

If the user has his/her hand 300 on a table (not shown) with the palm facing downwards towards the surface of the table, locations '3' and '4' may be used to detect heart pulse or heartbeat. If the fist is clenched with the arm lying on the table (not shown), location '2' may be more suitable for the detection of heart pulse or heartbeat. Other possible locations, such as, '1', '5' and '6' may also be used.

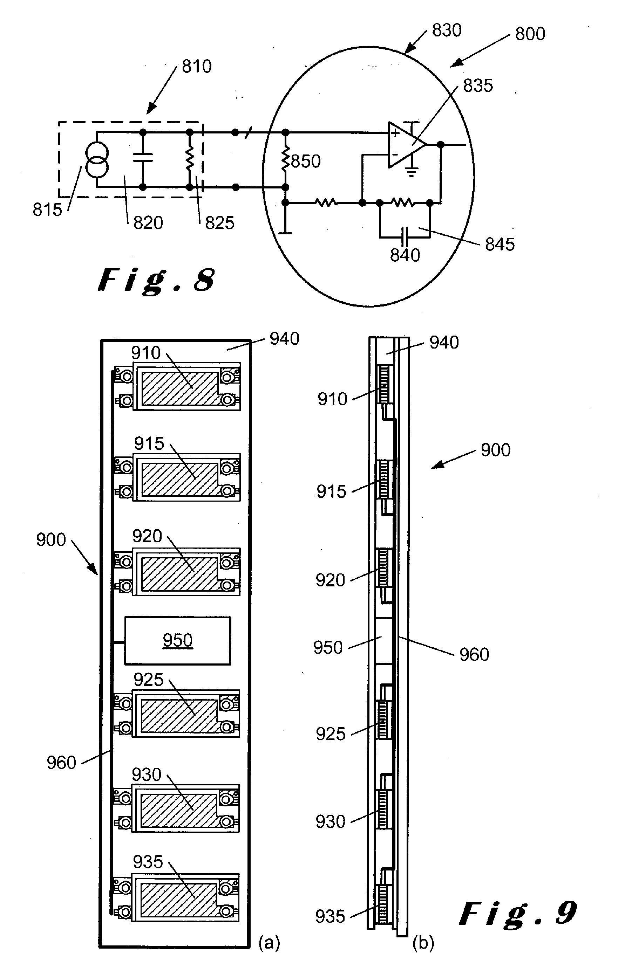

The orientation of the arm can change during sleep or sedentary activities, for example, sitting at a desk. Different orientations of the arm may influence the signal-to-noise ratio (SNR) at the indicated locations. Ideally, in order to allow for movement of the arm, and/or its orientation as well as the relative movement of the heart pulse monitoring device with respect to the pulse location, a plurality of sensors can be deployed at multiple locations, while trying to keep the complexity of the device as small as possible. Such an arrangement is described below with reference to Figures 9(a) and 9(b).

By making the sensors as small as possible and placing them in close proximity to one another, the movement information captured by both sensors increasingly correlates with one another. This is illustrated by Figure 4.

In Figure 4, a piezoelectric sensor 400 is shown that comprises a PVDF film 410 located between electrodes 420, 430. The charge, $Q_3$, accumulated between the electrodes 420, 430 is proportional to the electrode area (indicated as 440), the piezoelectric constant, $g_{33}$, and the average strain, $S_{average}$, in the thickness direction. The thickness direction is indicated by +3 to -3.

This is analytically expressed in the following equation:

$$Q_3 = A S_{average} g_{33} \Delta$$

were $\Delta$ is the thickness of the PVDF film.

The voltage, V, between the electrodes 420, 430, which forms the output signal of the sensor, is proportional to the charge, $Q_3$, and the capacitance, C, of the piezoelectric film where

$$V = \frac{Q_3}{C}$$

Hence, the smaller the sensor area, the smaller is the area over which the strain is integrated and the more local is the strain captured by the sensor.

Whilst trying to ensure that the motion signals from the primary sensor and the reference signal correlate as much as possible, it is also necessary to prevent the heart rate pulse or heartbeat signal energy reaching the reference sensor as adaptive filtering, if used, will remove both motion and heartbeat information during filtering.

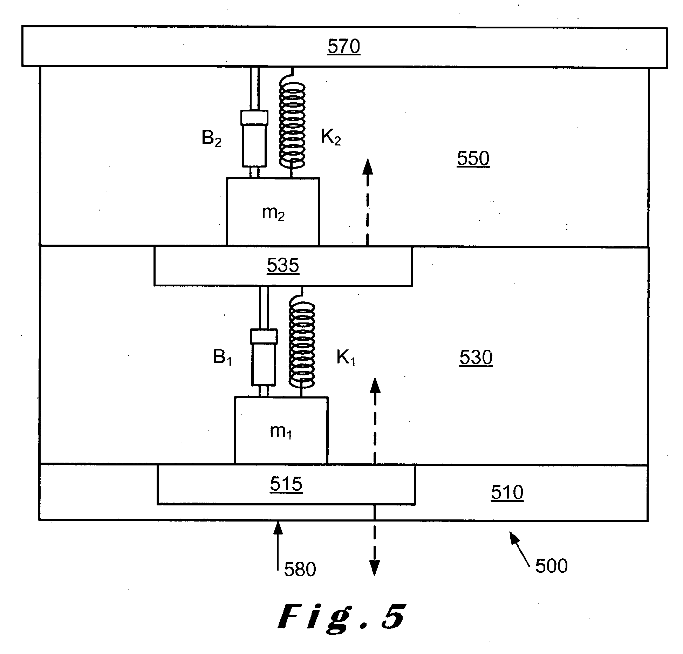

Figure 5 illustrates a schematic representation of a sensor package 500 in accordance with the present invention. The sensor package 500 comprises three packaging layers 510, 530, 550, each packaging layer having a different purpose. Packaging layer 510 contains a first sensor 515, the primary sensor, and is arranged to be located next to the skin of a user (not shown). Packaging layer 550 is arranged to be adjacent a strap 570 that holds the sensor package 500 in place against the wrist of the user to detect a pulse 580. Packaging layer 530 contains a second sensor 535, the reference or background sensor, and is located between the packaging layers 510 and 550. In this instance, both sensors 515 and 535 are piezoelectric sensors or transducers.

The material in packaging layers 530 and 550 can be represented, from the mechanical point of view, as respective mass-spring-damper arrangements. However, packaging layer 530 has a different purpose to that of packaging layer 550 as described above.

As shown in Figure 5, the material from which packaging layer 530 is made is defined by constants $m_1$, $B_1$, and $k_1$, where $m_1$ is the mass, $k_1$ is the spring constant, and $B_1$ is the damping constant of that particular material. Similarly, for packaging layer 550, the material from which it is made is defined by constants $m_2$, $B_2$, and $k_2$, where $m_2$ is the mass, $k_2$ is the spring constant, and $B_2$ is the damping constant of that particular material. These constants $m_1$, $m_2$, $k_1$, $k_2$, $B_1$ and $B_2$ are determined by the type of material and its specific dimensions within the sensor package 500. In each case, the transducer is assumed to have negligible mass due to its small thickness, for example, $40\mu m$.

In addition, the strap 570 may also be represented by a mass-spring-damper arrangement (not shown) where $m_3$ is the mass, $k_3$ is the spring, and $B_3$ is the damping constant. Here, these constants are determined by the material from which the strap 570 is made, the dimensions of the strap and its tightness against the wrist of the user.

The material of packaging layer 510 acts as an insulation layer between the skin of the wrist of a user and the piezoelectric sensor or transducer 515 and electrically isolates the skin from the first sensor 515. This insulation layer prevents signal distortion due to electrostatic coupling between the skin and the piezoelectric transducer 515. The piezoelectric sensor or transducer 515 corresponds to the first sensor, or primary sensor, described above with reference to Figure 1.

The material of packaging layer 530 serves to prevent the piezoelectric sensor or transducer 535, corresponding to the reference sensor as described above with reference to Figure 1, from being deformed due to heart pulse or heartbeat related vibrations. It effectively decouples the first sensor 515 from the second sensor 535. For this purpose, the material for packaging layer 530 may comprise θ, having a spring constant $k_1$ that is as small as possible in order to decouple mechanically the heart pulse or heartbeat vibration from the sensor 535. It will be appreciated that any other material can be used that provides the desired spring constant.

The material from which the packaging layer 550 is made is intended to provide a reaction force for the sensors 515 and 535 and allows both sensors 515, 535 to deform in accordance with detected pulse signal of the user, whilst isolating both the sensor 515, 535 from motion forces due to the strap 570. Depending on the material constants, sensors 515, 535 may be interchanged so that sensor 535 becomes the primary sensor and sensor 515 becomes the reference sensor as described above.

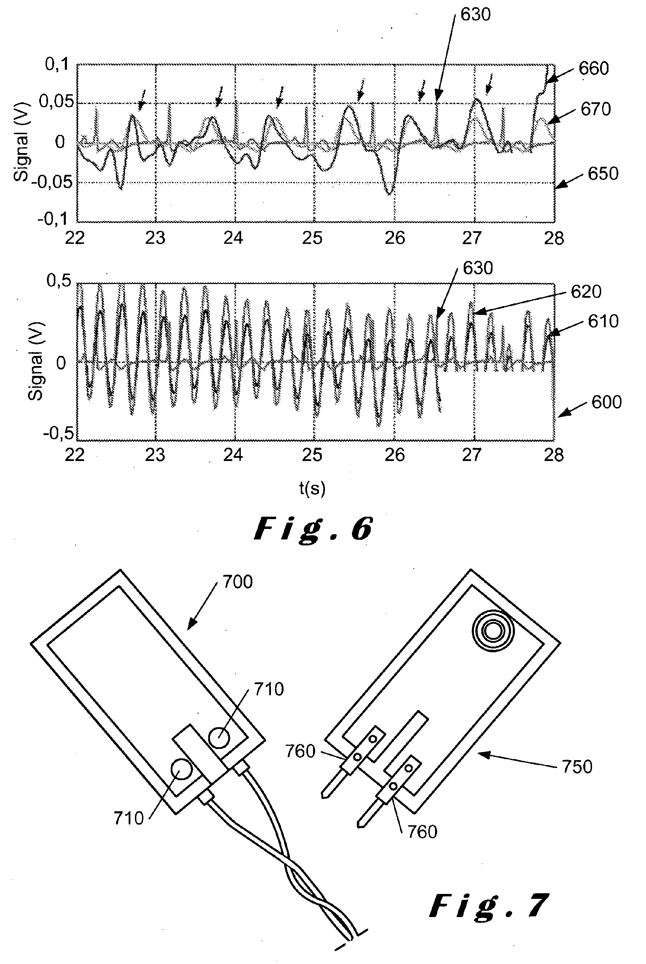

In cases where there is motion and both the primary and reference sensors detect artefact signals which correlate with one another, the artefact signal is cancelled and a heartbeat signal as shown in Figure 6.

In Figure 6, two plots 600, 650 are shown. In plot 600, signals obtained with the sensor package located at position '1' are shown for a specified period of time. Trace 610 refers to the first output signal from the primary sensor, trace 620 refers to the second output signal from the reference sensor, and trace 630 refers to an electrocardiogram (ECG) signal. All traces are indicated as voltage signals during that specified period of time.

In plot 650, for the same specified period, trace 660 refers to an adaptive filter output signal 660 and trace 670 refers to a template signal. Traces 660 and 670 are shown together with the ECG signal 630 from plot 600.

Motion artefacts are a problem in wrist-based heart rate measurement. A differential measurement with appropriate sensor packaging may eliminate motion artefact. In accordance with the present invention, the measurement system is effectively divided into four parts, namely, the sensor packaging, the lead attachments, the readout circuitry and the signal processing.

Signal processing is discussed first, as the requirements for the input signals place requirements on the rest of the system. Several signal processing algorithms may be used but each one needs to be compared for its ability to reduce motion artefact in a non-stationary environment. These signal processing algorithms include adaptive filtering, spatial averaging, and, independent and principal component analyses. It will be appreciated that each of these signal processing algorithms may be implemented separately or may be implemented as a combination in accordance with the particular application.

Principal and independent component analyses are examples of algorithms that take multiple data from several sensors as an input. Here, motion artefact and heartbeat signals have been mixed in unknown quantities. The output signals, referred to as source signals, are motion and heartbeat. In other words, a separation of the source signals is carried out, where the algorithm is blind to the amount of mixing of heartbeat and motion artefact.

In spatial averaging, signals from all primary sensors and reference sensors respectively can be summed up and divided to obtain two averaged signals. This averaging process may be weighted or non-weighted. In adaptive filtering, two signals are required with one acting as a primary and the other as a reference. The primary signal is supposed to contain signal plus noise, whereas the reference should only contain noise. A signal, s, is transmitted over a channel to a sensor that also receives a noise, $n_o$, uncorrelated with the signal. The combined signal and noise, $s + n_o$, form the primary input to a canceller. A second sensor receives a noise, $n_l$, uncorrelated with the signal but correlated in some unknown way with the noise, $n_o$. This sensor provides the reference input to the canceller. The noise $n_l$ is filtered to produce an output, $y$, that is as close a replica as possible of the noise, $n_o$. This output is subtracted from the primary input, $s + n_o$, to produce the system output or adaptive filtered signal, $e = s + n_o - y$, which should ideally only contain the heartbeat signal.

Noise and signal refer to motion- and heartbeat-induced vibration at the sensor location respectively. It is assumed that the signal and noise are statistically independent of each other and the noise in the reference input correlates strongly with the noise in the primary input. Other than this, no other knowledge is required about any of the input signals.

The adaptive filter assumes statistical independence between the signal and noise sources and a strong correlation between the noise signals in the reference and primary inputs respectively. The reference input should therefore only acquire the motion signal. Otherwise, a portion of the signal energy representing the heartbeat will also be removed by the adaptive filter, decreasing the SNR. The preferred approach to fulfil these requirements on the input signals is to decrease the sensor size, while packaging the primary and reference sensors appropriately. Note that the smaller sensor area allows both sensors to be placed closer to each other, ensuring better correlation of motion measured in both sensors, thereby better fulfilling the assumption of the adaptive filtering algorithm.

While different embodiments may contain different algorithms separately, a combination of the above algorithms may also be carried out, for example, spatial averaging followed by adaptive filtering or independent component analysis (ICA) on the spatially averaged signals to reduce the amount of data during processing.

Secondly, sensors need to be considered in relation to their type, location and packaging. A pair of mechanical sensors with a layer of mechanically isolating material located between the first and second sensors allow the reference and primary sensors to stay in close proximity to each other while ensuring that the reference sensor captures signals that only relate to movement.

As described above, piezoelectric polyvinyl difluoride (PVDF) film sensors with metal and polymer layers as packaging between the first and second sensors may be used. Several positions on the radial (thumb) side of the arm have been identified as possible locations to accommodate for the orientation of the arm in space. The packaging ensures that one sensor acts as a primary sensor by detecting both heart pulse rate or heartbeat related vibrations and motion artefacts, the second sensor acting as a reference sensor by only detecting motion of the user. The signals from these sensors are used in active noise cancellation involving an adaptive filter.

A wide variety of mechanical sensors exist. Piezoelectric sensors comprising PVDF as described above are one type of such a mechanical sensor. However, other materials, like nylon and polyvinyl chloride (PVC) also exhibit a similar mechanical effect. PVDF is biocompatible, can conform to the contours of the body, and possesses a high elastic compliance since it is a polymer. Its compliance and acoustic impedance matches that of human tissue, allowing heartbeat-related vibrations to be transferred effectively from the body of a user to the sensor.

Another approach using two different piezoelectric materials of acoustic impedances which match and mismatch the acoustic impedance of human tissue may also be used. In this case, the acoustic impedances of the two materials should be chosen so that they match each other as little as possible. Lead zirconate titanate (PZT) is an example of such a piezoelectric material that can be used for an acoustic mismatch situation. Such an approach can only be considered for body vibrations that have significantly higher frequencies, for example, in the range of a few tens of kHz.

The PVDF film used in the heart rate pulse monitor of the present invention is $75 \mu m$ thick. The PVDF film is coated with silver (metallised coating 220, 230 of Figure 2) which acts as an electrode for any wire or lead, which electrically connects the PVDF film to an analogue amplifier (located in the processing circuitry 150 of Figure 1). In certain commercial products, an additional protective layer (coating layer 240, 250 of Figure 2) is added to prevent mechanical damage to the metallised part or the PVDF sensor.

In relation to lead attachments, these allow the correlation of noise and prevent transfer of heartbeat signal energy to the reference sensor. A lead must be attached so that there is high electrical conductivity between the PVDF film and the lead itself so as to increase signal to noise ratio (SNR). The lead should have a low mass to avoid corruption of the signals due to mechanical vibration of the mass. In addition, a small volume may be desired so that the attachment does not interfere with heartbeat acquisition. Flexibility, mechanical strength and long-term stability are other features that may be selected appropriately.

A wide variety of attachment techniques exist. There are two commonly used methods, namely, penetrative and non-penetrative. Penetrative techniques involve using rivets, screws or crimp connectors which pierce through the film. Sometimes, an additional reinforcing laminate material is placed to increase contact strength. The metallisation on the film could be patterned or displaced such that the electrodes on both sides of the film do not effectively form a short circuit. Crimp connectors are known for their flexibility.

Non-penetrative methods involve low-melting point alloys such as tin, bismuth and indium or placing conductive rubber on either side of the film. Figure 7 shows examples of two kinds of lead attachments.

In Figure 7, a piezoelectric sensor 700 is shown that utilises rivets 710. A piezoelectric sensor 750 is also shown that utilises crimp connectors 760.

In an embodiment of the present invention, a piezoelectric sensor 700 with rivets for the lead attachments is utilised.

Piezoelectric sensors are self generating and do not require a current or voltage excitation. A piezoelectric sensor can, however, be modelled as a voltage or charge source, requiring respectively a voltage or a charge amplifier. Figure 8 illustrates a piezoelectric sensor arrangement 800 that is modelled as a charge source.

In Figure 8, the piezoelectric sensor arrangement 800 comprises a piezoelectric sensor 810 and a signal conditioning circuit 830. The sensor 810 can be represented as a charge source 815 coupled in parallel to a shunt capacitor 820 and a resistor 825. Alternatively, not shown, the sensor 810 can be represented as a voltage source with a series capacitor and resistor.

The signal conditioning circuit 830 comprises a non-inverting amplifier circuit 835 with a DC biasing is used to amplify the sensor output signal. A capacitor 840, placed in parallel with one of the amplifying resistors 845, results in a 1st order low pass filter which reduces electromagnetic interference. Resistor 850 provides a DC bias path for the single supply amplifier input stage 835 as shown.

In accordance with the present invention, the device is packaged to be worn at the wrist. In Figures 9(a) and 9(b), an array 900 of sensor packages 910, 915, 920, 925, 930, 935 as described above are shown attached to a wristlet 940. Figure 9(a) illustrates a bottom view and Figure 9(b) a side view. The wristlet 940 includes a housing 950 that contains a readout circuit (not shown) for both primary and reference sensors (also not shown) located in the sensor packages 910, 915, 920, 925, 930, 935.

It will readily be understood that the housing 950 also contains processing circuitry (not shown) for processing the signals provided by the primary and reference sensors in each of the sensor packages 910, 915, 920, 925, 930, 935. Connections between each sensor package 910, 915, 920, 925, 930, 935 and the housing 950 are shown by a communication bus 960 over which signals can be transmitted from each package 910, 915, 920, 925, 930, 935 to the housing 950.

In one embodiment of the present invention, the signals output from the primary and reference sensors comprise analogue signals. These analogue signals are converted into digital signals for transmission via a wireless link to, for example, an external display or a base station.

In another embodiment, in order to have good contact between sensors and skin so that the quality of the signals is maintained during ambulatory monitoring as well as comfort of the wearer, a piece of foam is placed between the reference sensor and the wrist band if the reference sensor is the sensor nearest to the wrist band.

## Claims

1. A heart pulse monitoring device (110; 500) for determining the heart pulse of a user, the heart pulse monitoring device comprising:-
   at least one sensor package (500) mountable over a pulse location of the user, each sensor package including a first sensor element (120; 515) and a second sensor element (130; 535), the first sensor element providing a first output signal (125) indicative of the heart pulse of the user and the second sensor element providing a second output signal (135) indicative of noise present in the first output signal, the first and second sensor elements comprise mechanical sensor elements mounted one on top of the other with a mechanically isolating material (140; 530) located between the first and second sensor elements, the mechanically isolating material isolating the first sensor element from the second sensor element;
   processing circuitry (150) connected to each sensor package, the processing circuitry being operable to process the first and second output signals to provide a processed output signal (155) indicative of the heart pulse of the user;
   **characterised in that** the first sensor element is mounted in a first packaging layer (510) and the second sensor element is mounted in a second packaging layer (530), the properties of each packaging layers being different to one another;
   and **in that** the first packaging layer is adapted to electrically isolate the skin from the first sensor element, and the second packaging layer is adapted to prevent the second sensor element from being deformed due to heart pulse or heartbeat related vibrations.

2. A heart pulse monitoring device according to claim 1, wherein one of the first and second mechanical sensor elements comprises a primary sensor element and the other of the first and second sensor elements comprises a reference sensor element, the reference sensor element being mechanically isolated from the primary sensor element by the mechanically isolating material.

3. A heart pulse monitoring device according to claim 1 or 2, wherein the material in at least one packaging layer can be represented as a mass-spring-damper arrangement.

4. A heart pulse monitoring device according to any one of the preceding claims, wherein each of the first and second mechanical sensor elements comprises a piezoelectric sensor element.

5. A heart pulse monitoring device according to claim 4, wherein each piezoelectric sensor element comprises a polyvinylidene fluoride film sensor element.

**6.** A heart pulse monitoring device according to claim 5, wherein the polyvinylidene fluoride film sensor element has an acoustic impedance that is matched to that of the user's tissue.

**7.** A heart pulse monitoring device according to claim 5, wherein one piezoelectric sensor element has an acoustic impedance that is matched to that of the user's tissue and the other piezoelectric sensor element has an acoustic impedance that is mismatched to that of the user's tissue.

**8.** A heart pulse monitoring device according to any one of the preceding claims, wherein the processing circuitry further includes a microprocessor in which the first and second output signals and the processed output signal are stored.

**9.** A heart pulse monitoring devi,ce according to any one of the preceding claims, wherein the processing circuitry further includes filtering circuitry for filtering the first and second output signals.

**10.** A heart pulse monitoring device according to any one of the preceding claims, wherein the processing circuitry further includes analogue-to-digital circuitry for converting the processed output signal into a digital output signal.

**11.** A heart pulse monitoring device according to any one of the preceding claims, further comprising a strap (570) for attaching the device to the user.

**12.** A heart pulse monitoring device according to claim 10, wherein the strap can be represented as a mass-spring-damper arrangement.

**13.** A heart pulse monitoring device according to claim 11, wherein the strap comprises a wristlet (940) and the device further comprises an array (900) of sensor packages (910, 915, 920, 925, 930, 935) arranged in the wristlet and connected to the processing circuitry.

**14.** A heart pulse monitoring device according to any one of the preceding claims, further comprising an oximeter for measuring oxygen levels in the blood of the user and for providing an oxygen level output signal to the processing circuitry.

**15.** A heart pulse monitoring device according to any one of the preceding claims, wherein the processing circuitry further includes wireless transmission equipment for transmitting at least the processed output signal to an external device.

**16.** A method for detecting a heart pulse of a user using a heart pulse monitoring device according to any one of the preceding claims, the method comprising the steps of:-

a) obtaining a first output signal (125) from the first sensor element (120; 515), the first output signal being indicative of the pulse of the user;
b) obtaining a second output signal (135) from the second sensor element (130; 535), the second output signal being indicative of noise in the first output signal;
c) removing noise from the first output signal using the second output signal; and
d) providing a processed output signal (155) that is representative of the pulse of the user.

**17.** A method according to claim 16, wherein the device comprises a plurality of sensor packages, and the method comprises, for each sensor package, executing steps a) to d).

**18.** A method according to claim 16 or 17, further comprising the step of transmitting data relating to the processed output signal for each sensor package to an external device.

**Patentansprüche**

**1.** Herzfrequenz-Überwachungsvorrichtung (110; 500) zum Bestimmen der Herzfrequenz eines Benutzers, wobei die Herzfrequenz-Überwachungsvorrichtung Folgendes umfasst:

mindestens ein Sensorgehäuse (500), das über einer Pulsstelle des Benutzers installierbar ist, wobei jedes Sensorgehäuse ein erstes Sensorelement (120; 515) und ein zweites Sensorelement (130; 535) umfasst, wobei das erste Sensorelement ein erstes Ausgangssignal (125) bereitstellt, das die Herzfrequenz des Benutzers angibt, und das zweite Sensorelement ein zweites Ausgangssignal (135) bereitstellt, das ein Rauschen angibt, das in dem ersten Ausgangssignal vorliegt, wobei die ersten und zweiten Sensorelemente mechanische Sensorelemente umfassen, die übereinander installiert sind, wobei sich ein mechanisch isolierendes Material (140; 530) zwischen den ersten und zweiten Sensorelementen befindet, wobei das mechanisch isolierende Material das erste Sensorelement von dem zweiten Sensorelement isoliert; Verarbeitungsschaltungen (150), die an jedem Sensorgehäuse angeschlossen sind, wobei die Verarbeitungsschaltungen betriebsfähig sind, um die ersten und zweiten Ausgangssignale zu verarbeiten, um ein verarbeitetes Ausgangssignal (155) bereitzustellen, das die Herzfrequenz des Benutzers angibt;

**dadurch gekennzeichnet, dass** das erste Sensorelement in einer ersten Gehäuseschicht (510) installiert ist und das zweite Sensorelement in einer zweiten Gehäuseschicht (530) installiert ist, wobei sich die Eigenschaften jeder Gehäuseschicht voneinander unterscheiden; und dass die erste Gehäuseschicht geeignet ist, um die Haut von dem ersten Sensorelement elektrisch zu isolieren, und die zweite Gehäuseschicht geeignet ist, um zu verhindern, dass das zweite Sensorelement auf Grund von Vibrationen in Zusammenhang mit der Herzfrequenz oder dem Herzschlag verformt wird.

2. Herzfrequenz-Überwachungsvorrichtung nach Anspruch 1, wobei eines von den ersten und zweiten mechanischen Sensorelementen ein primäres Sensorelement umfasst und das andere von den ersten und zweiten Sensorelementen ein Referenzsensorelement umfasst, wobei das Referenzsensorelement von dem primären Sensorelement durch das mechanisch isolierende Material mechanisch isoliert ist.

3. Herzfrequenz-Überwachungsvorrichtung nach Anspruch 1 oder 2, wobei das Material in mindestens einer Gehäuseschicht als Masse-Feder-Dämpfer-Anordnung dargestellt sein kann.

4. Herzfrequenz-Überwachungsvorrichtung nach einem der vorhergehenden Ansprüche, wobei jedes von den ersten und zweiten mechanischen Sensorelementen ein piezoelektrisches Sensorelement umfasst.

5. Herzfrequenz-Überwachungsvorrichtung nach Anspruch 4, wobei jedes piezoelektrische Sensorelement ein Sensorelement aus Polyvinylidenfluorid-Folie umfasst.

6. Herzfrequenz-Überwachungsvorrichtung nach Anspruch 5, wobei das Sensorelement aus Polyvinylidenfluorid-Folie eine akustische Impedanz aufweist, die derjenigen des Gewebes des Benutzers entspricht.

7. Herzfrequenz-Überwachungsvorrichtung nach Anspruch 5, wobei ein piezoelektrisches Sensorelement eine akustische Impedanz aufweist, die derjenigen des Gewebes des Benutzers entspricht, und das andere piezoelektrische Sensorelement eine akustische Impedanz aufweist, die nicht derjenigen des Gewebes des Benutzers entspricht.

8. Herzfrequenz-Überwachungsvorrichtung nach einem der vorhergehenden Ansprüche, wobei die Verarbeitungsschaltungen ferner einen Mikroprozessor umfassen, in dem die ersten und zweiten Ausgangssignale und das verarbeitete Ausgangssignal gespeichert sind.

9. Herzfrequenz-Überwachungsvorrichtung nach einem der vorhergehenden Ansprüche, wobei die Verarbeitungsschaltungen ferner Filterschaltungen umfassen, um die ersten und zweiten Ausgangssignale zu filtern.

10. Herzfrequenz-Überwachungsvorrichtung nach einem der vorhergehenden Ansprüche, wobei die Verarbeitungsschaltungen ferner Analog/Digital-Schaltungen umfassen, um das verarbeitete Ausgangssignal in ein digitales Ausgangssignal umzuwandeln.

11. Herzfrequenz-Überwachungsvorrichtung nach einem der vorhergehenden Ansprüche, ferner umfassend einen Riemen (570), um die Vorrichtung an dem Benutzer anzubringen.

12. Herzfrequenz-Überwachungsvorrichtung nach Anspruch 10, wobei der Riemen als Masse-Feder-Dämpfer-Anordnung dargestellt sein kann.

13. Herzfrequenz-Überwachungsvorrichtung nach Anspruch 11, wobei der Riemen ein Armband (940) umfasst und die Vorrichtung ferner eine Aufstellung (900) von Sensorgehäusen (910, 915, 920, 925, 930, 935) umfasst, die in dem Armband angeordnet sind und an die Verarbeitungsschaltungen angeschlossen sind.

14. Herzfrequenz-Überwachungsvorrichtung nach einem der vorhergehenden Ansprüche, ferner umfassend ein Oxymeter zum Messen des Sauerstoffniveaus im Blut des Benutzers und zum Bereitstellen eines Sauerstoffniveau-Ausgangssignals für die Verarbeitungsschaltungen.

15. Herzfrequenz-Überwachungsvorrichtung nach einem der vorhergehenden Ansprüche, wobei die Verarbeitungsschaltungen ferner eine drahtlose Übertragungseinrichtung zum Übertragen mindestens des verarbeiteten Ausgangssignals an eine externe Vorrichtung umfassen.

16. Verfahren zum Erkennen einer Herzfrequenz eines Benutzers unter Verwendung einer Herzfrequenz-Überwachungsvorrichtung nach einem der vorhergehenden Ansprüche, wobei das Verfahren folgende Schritte umfasst:

a) Erzielen eines ersten Ausgangssignals (125) von dem ersten Sensorelement (120; 515), wobei das erste Ausgangssignal den Puls des Benutzers angibt;
b) Erzielen eines zweiten Ausgangssignals (135) von dem zweiten Sensorelement (130;

535), wobei das zweite Ausgangssignal ein Rauschen in dem ersten Ausgangssignal angibt;

c) Unterdrücken des Rauschens aus dem ersten Ausgangssignal unter Verwendung des zweiten Ausgangssignals; und

d) Bereitstellen eines verarbeiteten Ausgangssignals (155), das den Puls des Benutzers darstellt.

17. Verfahren nach Anspruch 16, wobei die Vorrichtung eine Vielzahl von Sensorgehäusen umfasst, und das Verfahren für jedes Sensorgehäuse das Ausführen der Schritte a) bis d) umfasst.

18. Verfahren nach Anspruch 16 oder 17, ferner umfassend den Schritt des Übertragens von Daten bezüglich des verarbeiteten Ausgangssignals für jedes Sensorgehäuse an eine externe Vorrichtung.

**Revendications**

1. Dispositif de surveillance des pulsations du coeur (110 ; 500) pour déterminer le pouls cardiaque d'un utilisateur, le dispositif de surveillance des pulsations du coeur comprenant :

   au moins un boîtier de capteur (500) pouvant être monté sur un emplacement du pouls de l'utilisateur, chaque boîtier de capteur comprenant un premier élément capteur (120 ; 515) et un deuxième élément capteur (130 ; 535), le premier élément capteur fournissant un premier signal de sortie (125) indicateur de la fréquence cardiaque de l'utilisateur et le deuxième élément capteur fournissant un deuxième signal de sortie (135) indicateur du bruit présent dans le premier signal de sortie, les premier et deuxième éléments capteurs comprenant des éléments capteurs mécaniques montés l'un sur l'autre avec un matériau d'isolation mécanique (140 ; 530) situé entre les premier et deuxième éléments capteurs, le matériau d'isolation mécanique isolant le premier élément capteur du deuxième élément capteur ;

   une circuiterie de traitement (150) connectée à chaque boîtier de capteur, la circuiterie de traitement pouvant fonctionner pour traiter les premier et deuxième signaux de sortie pour fournir un signal de sortie traité (155) indicateur du pouls cardiaque de l'utilisateur ;

   **caractérisé en ce que** le premier élément capteur est monté dans une première couche d'emballage (510) et le deuxième élément capteur est monté dans une deuxième couche d'emballage (530), les propriétés de chaque couche d'emballage étant différentes les unes des autres ;

   et **en ce que** la première couche d'emballage est apte à isoler électriquement la peau du premier élément capteur et la deuxième couche d'emballage est apte à empêcher le deuxième élément capteur d'être déformé du fait des vibrations liées au pouls cardiaque ou aux battements du coeur.

2. Dispositif de surveillance des pulsations du coeur selon la revendication 1, dans lequel un des premier et deuxième éléments capteurs mécaniques comprend un élément capteur primaire et l'autre des premier et deuxième éléments capteurs comprend un élément capteur de référence, l'élément capteur de référence étant isolé mécaniquement de l'élément capteur primaire par le matériau d'isolation mécaniquement.

3. Dispositif de surveillance des pulsations du coeur selon la revendication 1 ou 2, dans lequel le matériau dans au moins une couche d'emballage peut être représenté comme un agencement masse-ressort-amortisseur.

4. Dispositif de surveillance des pulsations du coeur selon l'une quelconque des revendications précédentes, dans lequel chacun des premier et deuxième éléments capteurs mécaniques comprend un élément capteur piézoélectrique.

5. Dispositif de surveillance des pulsations du coeur selon la revendication 4, dans lequel chaque élément capteur piézoélectrique comprend un élément capteur sous forme de film de fluorure de polyvinylidène.

6. Dispositif de surveillance des pulsations du coeur selon la revendication 5, dans lequel l'élément capteur sous forme de film de fluorure de polyvinylidène a une impédance acoustique qui correspond à celle du tissu de l'utilisateur.

7. Dispositif de surveillance des pulsations du coeur selon la revendication 5, dans lequel un élément capteur piézoélectrique a une impédance acoustique qui correspond à celle du tissu de l'utilisateur et l'autre élément capteur piézoélectrique a une impédance acoustique qui ne correspond pas à celle du tissu de l'utilisateur.

8. Dispositif de surveillance des pulsations du coeur selon l'une quelconque des revendications précédentes, dans lequel la circuiterie de traitement comprend en outre un microprocesseur dans lequel les premier et deuxième signaux de sortie et le signal de sortie traité sont stockés.

**9.** Dispositif de surveillance des pulsations du coeur selon l'une quelconque des revendications précédentes, dans lequel la circuiterie de traitement comprend en outre une circuiterie de filtrage pour filtrer les premier et deuxième signaux de sortie.

**10.** Dispositif de surveillance des pulsations du coeur selon l'une quelconque des revendications précédentes, dans lequel la circuiterie de traitement comprend en outre une circuiterie analogique à numérique pour convertir le signal de sortie traité en un signal de sortie numérique.

**11.** Dispositif de surveillance des pulsations du coeur selon l'une quelconque des revendications précédentes, comprenant en outre une sangle (570) pour attacher le dispositif à l'utilisateur.

**12.** Dispositif de surveillance des pulsations du coeur selon la revendication 10, dans lequel la sangle peut être représentée comme un agencement masse-ressort-amortisseur.

**13.** Dispositif de surveillance des pulsations du coeur selon la revendication 11, dans lequel la sangle comprend un serre-poignet (940) et le dispositif comprend en outre un ensemble (900) de boîtiers de capteur (910, 915, 920, 925, 930, 935) agencés dans le serre-poignet et connectés à la circuiterie de traitement.

**14.** Dispositif de surveillance des pulsations du coeur selon l'une quelconque des revendications précédentes, comprenant en outre un oxymètre pour mesurer les niveaux d'oxygène dans le sang de l'utilisateur et pour fournir un signal de sortie de niveau d'oxygène à la circuiterie de traitement.

**15.** Dispositif de surveillance des pulsations du coeur selon l'une quelconque des revendications précédentes, dans lequel la circuiterie de traitement comprend en outre un équipement de transmission sans fil pour transmettre au moins le signal de sortie traité à un dispositif externe.

**16.** Procédé de détection d'un pouls cardiaque d'un utilisateur utilisant un dispositif de surveillance des pulsations du coeur selon l'une quelconque des revendications précédentes, le procédé comprenant les étapes consistant à :

a) obtenir un premier signal de sortie (125) du premier élément capteur (120 ; 515), le premier signal de sortie étant indicateur du pouls de l'utilisateur ;
b) obtenir un deuxième signal de sortie (135) du deuxième élément capteur (130 ; 535), le deuxième signal de sortie étant indicateur de bruit dans le premier signal de sortie ;
c) éliminer le bruit du premier signal de sortie en utilisant le deuxième signal de sortie ; et
d) fournir un signal de sortie traité (155) qui est représentatif du pouls de l'utilisateur.

**17.** Procédé selon la revendication 16, dans lequel le dispositif comprend une pluralité de boîtiers de capteur, et le procédé comprend, pour chaque boîtier de capteur, l'exécution des étapes a) à d).

**18.** Procédé selon la revendication 16 ou 17, comprenant en outre l'étape de transmission de données relatives au signal de sortie traité pour chaque boîtier de capteur à un dispositif externe.

**Fig. 1**

**Fig. 2**

**Fig. 3**

**Fig. 4**

*Fig.5*

*Fig. 6*

*Fig. 7*

*Fig. 8*

(a)　(b)　*Fig. 9*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5807267 A

- US 6491647 B

**Non-patent literature cited in the description**

- Pulse Wave Sensor for Non-intrusive Driver's Drowsiness Detection. *31st Annual International Conference of the IEEE EMBS Minneapolis, Minneapolis USA,* 02 September 2009